(19) Europäisches Patentamt
European Patent Office
Office européen des brevets

(11) Veröffentlichungsnummer : **0 342 536 B1**

(12) # EUROPÄISCHE PATENTSCHRIFT

(45) Veröffentlichungstag der Patentschrift : **23.09.92 Patentblatt 92/39**

(21) Anmeldenummer : **89108550.8**

(22) Anmeldetag : **12.05.89**

(51) Int. Cl.$^5$ : **C07D 409/04,** // A61K31/50 , (C07D409/04, 333:00, 241:00)

(54) **Thienyl-Piperazinone, ihre Herstellung und Verwendung.**

(30) Priorität : **20.05.88 DE 3817198**

(43) Veröffentlichungstag der Anmeldung : **23.11.89 Patentblatt 89/47**

(45) Bekanntmachung des Hinweises auf die Patenterteilung : **23.09.92 Patentblatt 92/39**

(84) Benannte Vertragsstaaten : **AT BE CH DE ES FR GB GR IT LI NL SE**

(56) Entgegenhaltungen :
**EP-A- 0 072 932**
**DE-A- 1 197 089**
**US-A- 2 653 153**

(73) Patentinhaber : **CASSELLA Aktiengesellschaft**
**Hanauer Landstrasse 526**
**W-6000 Frankfurt am Main 61 (DE)**

(72) Erfinder : **Schönafinger, Karl, Dr.**
**Holunderweg 8,**
**d-8755 Alzenau (DE)**
Erfinder : **Beyerle, Rudi, Dr.**
**An der Pfaffenmauer 44**
**W-6000 Frankfurt/Main 60 (DE)**
Erfinder : **Schindler, Ursula, Dr.**
**Reinhardswaldweg 1**
**W-6082 Mörfelden-Walldorf 2 (DE)**

(74) Vertreter : **Urbach, Hans-Georg, Dr. et al**
**Hanauer Landstrasse 526**
**W-6000 Frankfurt am Main 61 (DE)**

EP 0 342 536 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft in 3-Stellung substituierte 3-(Thien-2-yl)-piperazin-2-one der allgemeinen Formel I

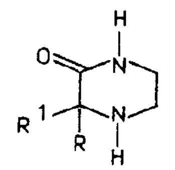

( I )

worin

R Phenyl; Phenyl mono-, di- oder tri-substituiert durch $(C_1-C_6)$Alkyl, $(C_1-C_6)$Alkoxy und/oder $(C_1-C_6)$Alkoxy-$(C_1-C_6)$alkyl; $(C_1-C_6)$Alkyl; Phenyl-$(C_1-C_6)$alkyl; Naphthyl-$(C_1-C_6)$alkyl; $(C_1-C_6)$Alkoxy-$(C_1-C_6)$alkyl; Di-$(C_1-C_6)$alkylamino-$(C_1-C_6)$alkyl und

$R^1$ 2-Thienyl

bedeuten, sowie ihre pharmakologisch verträglichen Säureadditionsverbindungen.

Die Alkyl- und Alkoxyreste können, auch wenn sie als Substituenten anderer Reste auftreten, unverzweigt oder verzweigt sein und besitzen vorzugsweise 1 bis 4 C-Atome. Der für R stehende substituierte Phenylrest ist vorzugsweise monosubstituiert, insbesondere in 2- und 4-Stellung.

Beispiele für geeignete Reste R sind:

Methyl; Ethyl; Isopropyl; Propyl; Butyl; sec.Butyl; Isobutyl; tert.-Butyl; Pentyl; Hexyl; Isohexyl; 2-Methoxyethyl; 2-Ethoxyethyl; 2-Propoxyethyl; 2-Butoxyethyl; 2-Hexyloxyethyl; 3-Methoxypropyl; 3-Ethoxypropyl; 3-Propoxypropyl, 2-Methoxypropyl; 2-Ethoxypropyl; 2-Propoxypropyl; 3- oder 4-Methoxybutyl; 3- oder 4-Propoxybutyl; 3- oder 4-Isopropoxybutyl; 3- oder 4-Butoxybutyl; 3-, 4- oder 5-Methoxypentyl; 3-, 4- oder 5-Ethoxypentyl; 3-, 4- oder 5-Propoxypentyl; 3-, 4-, 5-oder 6-Methoxyhexyl; 2-Dimethylamino-ethyl; 2-Diethylamino-ethyl; 2-Dibutylamino-ethyl; 2-Dihexylamino-ethyl; 2- oder 3-Dimethylamino-propyl; 2- oder 3-Diethylamino-propyl; 2- oder 3-Dipropylamino-propyl; 2-, 3- oder 4-Dimethylamino-butyl; 2-, 3- oder 4-Diethylamino-butyl; 2-, 3- oder 4-Dibutylamino-butyl; Phenyl; 2-, 3- oder 4-Methyl-, -Ethyl-, -Propyl-, -Isopropyl-, -Butyl-, -Hexyl-phenyl; 2-, 3- oder 4-Methoxy-, -Ethoxy-, -Propoxy-, -Isopropoxy-, -Butoxy-, -Pentyloxy-phenyl; 2-, 3- oder 4-(2-Methoxyethyl)-phenyl; 2-, 3- oder 4-(2-Ethoxyethyl)-phenyl; 2-, 3- oder 4-(2-Butoxyethyl)-phenyl; 2-, 3- oder 4-(3-Methoxypropyl)-phenyl; 2-, 3- oder 4-(3-Ethoxypropyl)-phenyl; 2-, 3- oder 4-(3-Butoxypropyl)-phenyl; 2,3-, 2,4-, 2,5-, 2,6-, 3,4-oder 3,5-Di-methyl-, -ethyl-, -propyl- oder -butyl-phenyl; 2,3-, 2,4-, 2,5-, 2,6-, 3,4- oder 3,5-Dimethoxy, -ethoxy-, -propoxy-, -isopropoxy-oder -butoxy-phenyl; 2,3,4-, 2,3,5-, 2,3,6-, 2,4,5-, 2,4,6-, 2,5,6- oder 3,4,5-Tri-methyl-, -ethyl-, -propyl-, -butyl-, -methoxy-, -ethoxy-, -butoxy-phenyl; Benzyl; 2-Phenethyl; 3-Phenylpropyl; 2-(α- oder ß-Naphthyl)-ethyl.

Bevorzugte Beispiele für R sind Methyl, Ethyl, Isopropyl, Phenyl, Benzyl, Phenethyl; besonders bevorzugt ist Methyl.

Die Verbindungen der allgemeinen Formel lassen sich dadurch herstellen, daß das 3-(Thien-2-yl)-5,6-dihydro-(1H)-pyrazin-2-on der Formel II

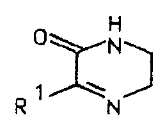

( I I )

mit einer metallorganischen Verbindung der allgemeinen Formel III

R - M (III)

worin M die Bedeutung von Li, $(CuLi)_{0,5}$, $Cd_{0,5}$, ZnHal, MgHal (Hal = I, Br oder Cl) hat und R die bereits angegebene Bedeutung besitzt, umgesetzt wird.

Die metallorganischen Verbindungen der Formel III können in an sich bekannter Weise hergestellt werden. Grignard-Verbindungen RMgHal, zinkorganische Verbindungen RZnHal und lithiumorganische Verbindungen RLi werden in an sich bekannter Weise durch Umsetzung von Halogenverbindungen der allgemeinen Formel IV

RHal (IV)

mit Mg, Zn oder Li in einem wasserfreien organischen Lösungsmittel, z.B. einem Ether oder einem aliphatischen

Kohlenwasserstoff hergestellt. Zum Teil sind sie auch handelsüblich, wie z.B. Methyllithium, n-, sec- und tert-Butyllithium. Cadmiumorganische Verbindungen $R_2Cd$ kann man aus Grignard-Verbindungen RMgBr durch Ummetallierung mit Cadmiumbromid $CdBr_2$ erhalten. Kupferlithiumverbindungen $R_2CuLi$ kann man aus Lithiumverbindungen RLi in Ether nach folgendem Schema herstellen:

$$CuI + RLi \xrightarrow[-Li]{} RCu \xrightarrow{RLi} R_2CuLi$$

Für Hal ist I oder Br bevorzugt. Von den metallorganischen Verbindungen III sind die Grignard-Verbindungen RMgHal wegen ihrer leichten Zugänglichkeit und Handhabbarkeit bevorzugt.

Die Umsetzung zwischen der Verbindung der Formel II und der Verbindung der allgemeinen Formel III wird normalerweise in einem inerten organischen Lösungsmittel bzw. Dispergiermittel durchgeführt. Geeignete inerte Lösungsmittel sind z.B. Ether, insbesondere solche mit 2 bis 8 C-Atomen im Molekül, wie z.B. Diethylether, Methyl-ethyl-ether, Di-n-propyl-ether, Di-iso-propyl-ether, Methyl-n-butyl-ether, Ethylpropylether, Di-butyl-ether, Tetrahydrofuran; 1,4-Dioxan, 1,2-Dimethoxyethan, Bis-β-methoxyethyl-ether; Oligoethylen-glykol-dimethyl-ether, wie z.B. Pentaglyme; aliphatische Kohlenwasserstoffe, wie z.B. Pentan, Hexan, Heptan, Octan, niedrig- und hochsiedende Petrolether; aromatische Kohlenwasserstoffe, wie z.B. Benzol, Toluol, o-, m- und p-Xylol. Auch Gemische verschiedener inerter Lösungsmittel können verwendet werden.

Die Umsetzung wird, wie bei Reaktionen mit Grignardreagenzien oder anderen metallorganischen Verbindungen üblich, zweckmäßigerweise unter Ausschluß von Feuchtigkeit und insbesondere bei länger dauernden Umsetzungen unter einem Inertgas durchgeführt. Geeignete Inertgase sind. z.B. Edelgase, wie Argon, und Stickstoff.

Die Reaktionstemperatur kann in weiten Bereichen variieren. Im allgemeinen erfolgt die Umsetzung im Temperaturbereich von 0°C bis zur Siedetemperatur des verwendeten Lösungsmittels oder Lösungsmittelgemischs. Die Reaktion wird in vielen Fällen bei Temperaturen von 0 bis 50°C, vorzugsweise 15 bis 40°C, durchgeführt.

Normalerweise erfolgt die Umsetzung bei Normaldruck, kann aber auch bei einem vom Normaldruck abweichenden Druck durchgeführt werden.

Die Aufarbeitung der Reaktionsansätze erfolgt normalerweise so, daß zunächst durch Zugabe von Wasser hydrolysiert und die gewünschte Verbindung dann in üblicher Weise isoliert wird.

Die Verbindungen der Formel I können in Säureadditionssalze überführt werden. Zur Bildung derartiger Säureadditionssalze sind anorganische und organische Säuren geeignet. Geeignete Säuren sind beispielsweise Chlorwasserstoff, Bromwasserstoff, Naphthalindisulfonsäure, insbesondere 1,5-Naphthalindisulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Citronen-oder Adipin-Säure. Pharmakologisch annehmbare Säuradditionssalze werden bevorzugt. Die Säureadditionssalze können wie üblich durch Vereinigung der Verbindung I und der Säure, zweckmäßigerweise in einem geeigneten Lösungs-oder Verdünnungsmittel, hergestellt werden. Geeignete Lösungs- oder Verdünnungsmittel sind z.B. organische Lösungsmittel, wie Alkohole, insbesondere solche mit 1 bis 6 C-Atomen, wie z.B. Methanol, Ethanol, i-Propanol, oder die bereits genannten Ether, ferner Ester, Ketone etc.

Das als Ausgangsprodukt benötigte 3-(Thien-2-yl)-5,6-dihydro-(1H)-pyrazin-2-on der Formel I ist aus Beispiel 42 der USP 3 056 784 bekannt.

Die zur Herstellung von Verbindungen der Formel III benötigten Ausgangsverbindungen der Formeln IV sind bekannt oder können leicht nach den für die jeweilige Verbindungsklasse bekannten Verfahren hergestellt werden.

Die erfindungsgemäßen Piperazinone der Formel I und ihre pharmakologisch verträglichen Salze sind Nootropika, d.h. sie dienen zur Behandlung von menschlichen Krankheiten, die durch eine Einschränkung der Gehirnfunktion, insbesondere der Gedächtnisleistung, charakterisiert sind, sowie zur Minderung der Folgen cerebraler Alterungsvorgänge.

Sie sind bekannten Verbindungen ähnlicher Wirkungsrichtung in ihrer Wirksamkeit überlegen. Gegenüber der Verbindung des Beispiels 16 der EP-A2-72 932 zeigen sie ein anderes Wirkprofil bei gleichzeitiger hervorragender Wirksamkeit.

Die erfindungsgemäßen Verbindungen der Formel I und/oder ihre pharmakologisch verträglichen Säureadditionsverbindungen können am Menschen als Heilmitel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis einer erfindungsgemäßen Verbindung und-

/oder eines ihrer Salze neben üblichen pharmazeutisch einwandfreien Träger- und Zusatzstoffen enthalten. Die Zubereitungen enthalten normalerweise etwa 0,5 bis 90 Gew.% der erfindungsgemäßen Wirkstoffe.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden.

Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, verschiedene Stärken, Stärke oder Derivate davon, wie z.B. Stärkehydrolysate, Calciumphosphate, Zellulose und Zellulosederivate, Milchzucker, Hexite, Siliciumdioxid, Talk, Stearinsäure oder deren Salze etc. verwenden. Trägerstoffe für Weichgelatinekapseln und Suppositorien sind z.B. Fette, Wachse, synthetische Polymere, halbfeste und flüssige Polyole, natürliche oder gehärtete Öle etc.Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, mikrobiologisch aktive Verbindungen, wie z.B. Konservierungsmittel oder Antiseptica, ferner Lösungsmittel oder Lösungsvermittler oder Mittel zur Erzielung eines Depoteffekts, sowie Salze zur Veränderung des osmotischen Drucks, Überzugsmittel oder Antioxidantien in den üblichen Konzentrationen enthalten.

Die pharmazeutischen Präparate können neben einer oder mehreren Verbindungen der allgemeinen Formel I auch noch eine oder mehrere andere pharmazeutisch wirksame Substanzen, beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbocromen, Dipyridamol, Nifedipin und Perhexilin; antianginöse Verbindungen, wie Isosorbiddinitrat, Isosorbidmononitrat, Glyceroltrinitrat, Molsidomin und Verapamil;
ß-Blocker, wie Propranolol, Oxprenolol, Atenolol, Metoprolol und Penbutolol und oogen-metabolische Mittel, wie Pirilinol. Darüber hinaus lassen sich die Verbindungen auch mit anderen nootrop wirkenden Substanzen, wie z.B. Piracetam und/oder Verbindungen der EP-A2-72 932 kombinieren.

Die geeigneten Dosen der Wirkstoffe können in weiten Grenzen variiert und so an die individuellen Erfordernisse des Einzelfalls angepaßt werden. In der Regel beträgt die Tagesdosis 0,1 bis 150 mg, vorzugsweise 1 bis 30 mg, pro Patient bei oraler Applikation. Auch bei anderweitiger Applikation liegen die Tagesdosen dank der guten Resorbierbarkeit der Wirkstoffe in dem gleichen Mengenbereich.

Trotzdem kann es gegebenenfalls erforderlich sein, von den genannten Mengen abzuweichen, und zwar in Abhängigkeit vom Körpergewicht bzw. der Art der Applikation, aber auch aufgrund des Zeitpunktes bzw. Intervalles, zu welchem die Verabreichung erfolgt. So kann es in einigen Fällen ausreichend sein, mit weniger als den vorgenannten Mengen zu dosieren.

Die Tagedosis kann auf einmal gegeben werden, wird jedoch in der Regel in mehreren, beispielsweise 2 bis 4 Teildosen verabreicht. Die Einzeldosis der wirksamen Substanz beträgt dabei in der Regel 0,001 bis 2 mg pro kg Körpergewicht. Pharmazeutische Präparate enthalten normalerweise 0,1 bis 50 mg, vorzugsweise 0,1 bis 10 mg, Wirkstoff der Formel I oder eines pharmakologisch annehmbaren Salzes pro Dosis.

Zum Nachweis der pharmakologischen Wirksamkeit der erfindungsgemäßen Verbindungen wurde die Natrium-Nitrit Hypoxie bei der Maus geprüft. In diesem Test wird nach der Methode von Gibson und Blass (J. Neurochem. 27, 37 (1976) bei Mäusen durch Verabreichung von Natrium-Nitrit (175 mg/kg s.c.) eine cerebrale Hypoxie erzeugt, die zu massiven Verhaltensstörungen der Tiere führt. Betimmt wird, ob die Haltefähigkeit an einem Drehstab durch Prämedikation mit der Testsubstanz beeinflußt wird. Die erfindungsgemäßen Verbindungen werden dabei in den Dosierungen 3 bzw. 30 mg/kg per os verabreicht. Die Ergebnisse sind in der nachfolgenden Tabelle zusammengefaßt.

## Tabelle

Prozentuale Umkehr der Störung der Haltefähigkeit nach Verabreichuung von Natrium-Nitrit und Vorbehandlung mit den Prüfsubstanzen der Formel I

| Verbindung Nr. | Substituent R | Dosis | % Umkehr |
|---|---|---|---|
| 1 | -phenyl | 30 mg/kg | 73 |
| 2 | -methyl | 30 mg/kg | 99 |
| 3 | -ethyl | 30 mg/kg | 55 |
| 4 | -(3,3-dimethylbutyl) | 3 mg/kg | 51 |
| 5 | -H | 30 mg/kg | 24 |

Die Verbindungen Nr. 1 bis 4 der vorstehenden Tabelle sind erfindungsgemäße Verbindungen. Bei der Verbindung der Nr. 5 der vorstehenden Tabelle handelt es sich um die Verbindung des Beispiels 16 der EP-A2-72932.

Die folgenden Ausführungsbeispiele 1 und 2 veranschaulichen die Herstellung der erfindungsgemäßen Verbindungen. Die Beispiele A bis E betreffen pharmazeutische Zubereitungen.

### Beispiel 1

3-Methyl-3-(thien-2-yl)-piperazin-2-on

Zur Lösung von 18 g 3-(Thien-2-yl)-5,6-dihydro-pyrazin-2-on in 150 ml Tetrahydrofuran wird die Grignard-Lösung, hergestellt aus 7,3 g Mg und 42,6 g Iodmethan in 150 ml Diethylether bei Raumtemperatur langsam zugetropft. Es wird 15 h bei Raumtemperatur nachgerührt und dann mit 100 ml Wasser hydrolysiert. Die Mischung wird mit conz. HCl auf pH = 2 gestellt, die organische Phase abgetrennt und die wäßrige Phase zweimal mit Diethylether ausgeschüttelt und mit Pottasche auf pH = 8 eingestellt. Die Verbindung wird nun mit Methylenchlorid extrahiert. Die Methylenchlorid-Phase wird getrocknet und eingeengt. Das zurückbleibende Öl wird durch Säulenchromatographie an Kieselgel der Firma Merck, Darmstadt, Type "0,04 - 0,06", mobile Phase : Methylenchlorid : Methanol im Volumenverhältnis 95 : 5 gereinigt. Die die Verbindungen enthaltenden Fraktionen werden im Rotationsverdampfer eingeengt und der Rückstand aus Isopropanol umkristallisiert.
Ausbeute: 8,0 g; Fp =113-116°C

### Beispiel 2

3-Phenyl-3-(thien-2-yl)-piperazin-2-on

Die Verbindung wird analog Beispiel 1 aus Brombenzol, Magnesium und 3-(Thien-2-yl)-4,5-dihydro-pyrazin-2-on erhalten.
Ausbeute: 17 g; Fp = 159-161°C (aus Isopropanol)

Das Hydrochlorid dieser Verbindung wird durch Lösen in Ethanol und Zufügen von mit HCl gesättigtem Essigester erhalten.
Fp = 215-218°C

Beispiel 3

3-Isopropyl-3-(thien-2-yl)-piperazin-2-on

Die Verbindung wird analog Beispiel 1 aus Isopropylchlorid, Magnesium und 3-(Thien-2-yl)-4,5-dihydro-pyrazin-2-on erhalten.

Die Herstellung des Grignard-Reagenzes und die eigentliche Umsetzung erfolgten in 1,4-Dioxan als Lösungsmittel.

Fp = 106-107°C

Beispiel 4

3-(3,3-Dimethylbutyl)-3-(thien-2-yl)-piperazin-2-on

Die Verbindung wird analog Beispiel 1 aus 3,3-Dimethylbutylchlorid, Magnesium und 3-(Thien-2-yl)-4,5-dihydro-pyrazin-2-on erhalten.

Die Herstellung des Grignard-Reagenzes und die eigentliche Umsetzung erfolgten in 1,2-Dimethoxyethan als Lösungsmittel.

Fp = 88-90°C

Beispiel 5

3-Ethyl-3-(thien-2-yl)-piperazin-2-on

Die Verbindung wird analog Beispiel 1 aus Ethylbromid, Magnesium und 3-(Thien-2-yl)-4,5-dihydro-pyrazin-2-on erhalten.

Die Herstellung des Grignard-Reagenzes und die eigentliche Umsetzung erfolgten in Diethylether als Lösungsmittel.

Fp = 68-70°C

Beispiel 6

3-(2-Methoxyethyl)-3-(thien-2-yl)-piperazin-2-on

Die Verbindung wird analog Beispiel 1 aus 2-Methoxyethylchlorid, Magnesium und 3-(Thien-2-yl)-4,5-dihydro-pyrazin-2-on erhalten.

Die Herstellung des Grignard-Reagenzes und die eigentliche Umsetzung erfolgten in Bis-β-methoxyethylether als Lösungsmittel.

Fp = 83-85°C

Beispiel 7

3-(2-Dimethylaminoethyl)-3-(thien-2-yl)-piperazin-2-on

Die Verbindung wird analog Beispiel 1 aus 2-Dimethylaminoethylchlorid, Magnesium und 3-(Thien-2-yl)-4,5-dihydro-pyrazin-2-on erhalten.

Die Herstellung des Grignard-Reagenzes und die eigentliche Umsetzung erfolgten in Tetrahydrofuran als Lösungsmittel.

Fp = 77-80°C

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | pro ml |
|---|---|
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

Emulsion, enthaltend 3 mg Wirkstoff pro 5 ml

| | pro 100 ml Emulsion |
|---|---|
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

Rektale Arzneiform, enthaltend 4 mg Wirkstoff pro Suppositorium

| | pro Suppositorium |
|---|---|
| Wirkstoff | 4 mg |
| Suppositoriengrundmasse | ad 2 g |

Beispiel E

Tabletten, enthaltend 2 mg Wirkstoff pro Tablette

| | pro Tablette |
|---|---|
| Wirkstoff | 20 mg |
| Maisstärke (weiß) | 30 mg |
| Milchzucker | 60 mg |
| Lösliche Stärke | 3 mg |
| Magnesiumstearat | 2 mg |
| | 115 mg |

Beispiel F

Tropfen (20 mg in 1 ml = 20 Tropfen)

| | |
|---|---|
| Wirkstoff | 2,00 g |
| Benzoesäuremethylester | 0,07 g |
| Benzoesäurethylester | 0,03 g |
| Ethanol 96%ig | 2 ml |
| entmineralisiertes Wasser | ad 100 ml |

## Patentansprüche

### Patentansprüche für folgende Vertragsstaaten : AT, BE, CH, DE, FR, GB, IT, LI, NL, SE

1. In 3-Stellung substituierte 3-(Thien-2-yl)-piperazin-2-one der allgemeinen Formel I

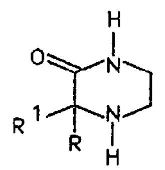

(I)

worin
R Phenyl; Phenyl mono-, di- oder tri-substituiert durch $(C_1-C_6)$Alkyl, $(C_1-C_6)$Alkoxy und/oder $(C_1-C_6)$Alkoxy-$(C_1-C_6)$alkyl; $(C_1-C_6)$Alkyl; Phenyl-$(C_1-C_6)$alkyl; Naphthyl-$(C_1-C_6)$alkyl; $(C_1-C_6)$Alkoxy-$(C_1-C_6)$alkyl; Di-$(C_1-C_6)$alkylamino-$(C_1-C_6)$alkyl und
$R^1$ 2-Thienyl
bedeuten, sowie ihre pharmakologisch verträglichen Säureadditionsverbindungen.

2. Piperazinone nach Anspruch 1, dadurch gekennzeichnet, daß R Phenyl; Phenyl mono-, di- oder tri-substituiert durch $(C_1-C_4)$Alkyl, $(C_1-C_4)$Alkoxy und/oder $(C_1-C_4)$Alkoxy-$(C_1-C_4)$alkyl; $(C_1-C_4)$Alkyl; $(C_1-C_4)$Alkoxy-$(C_1-C_6)$alkyl; Phenyl-$(C_1-C_4)$alkyl; Naphthyl-$(C_1-C_4)$alkyl; Di-$(C_1-C_4)$alkylamino-$(C_1-C_4)$alkyl bedeutet.

3. Piperazinone nach Anspruch 1 und/oder 2, dadurch gekennzeichnet, daß das für R stehende substituierte Phenyl monosubstituiert ist.

4. Piperazinone gemäß Anspruch 1, dadurch gekennzeichnet, daß R $(C_1-C_4)$Alkyl bedeutet.

5. Piperazinone gemäß Anspruch 1, dadurch gekennzeichnet, daß R Methyl, Ethyl, i-Propyl, Phenyl, Benzyl oder Phenethyl bedeutet.

6. 3-Methyl-3-(thien-2-yl)-piperazin-2-on gemäß Anspruch 1 und seine pharmakologisch verträglichen Säureadditionsverbindungen.

7. 3-Phenyl-3-(thien-2-yl)-piperazin-2-on gemäß Anspruch 1 und seine pharmakologisch verträglichen Säureadditionsverbindungen.

8. Verfahren zur Herstellung der in einem oder mehreren der Ansprüche 1 bis 7 angegebenen Piperazin-2-on-verbindungen, dadurch gekennzeichnet, daß das 3-(Thien-2-yl)-5,6-dihydro-(1H)-pyrazin-2-on der Formel II

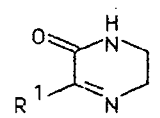

(II)

mit einer metallorganischen Verbindung der allgemeinen Formel III

R - M (III)

worin M die Bedeutung von Li, $(CuLi)_{0,5}$, $Cd_{0,5}$, ZnHal, MgHal (Hal = I, Br oder Cl) und R die bereits angegebene Bedeutung besitzen, in einem inerten organischen Lösungsmittel oder Lösungsmittelgemisch im Temperaturbereich von 0°C bis zum Siedepunkt des Lösungsmittels oder Lösungsmittelgemischs umgesetzt, in üblicher Weise aufgearbeitet und gegebenenfalls in eine pharmakologisch annehmbare Säureadditionsverbindung überführt wird.

9. Verwendung der Piperazinone der Ansprüche 1 bis 7 und/oder ihre pharmakologisch annehmbaren Säureadditions- verbindungen als pharmakologische Wirkstoffe zur Herstellung pharmazeutischer Zubereitungen, die zur Verbesserung der Gehirnfunktion des Menschens und/oder zur Minderung der Folgen cerebraler Alterungsvorgänge des Menschens bestimmt sind.

10. Pharmazeutisches Präparat, dadurch gekennzeichnet, daß es eine Verbindung der Ansprüche 1 bis 7 oder ein pharmakologisch annehmbares Säureadditionssalz davon als Wirkstoff in einer pharmakologisch wirksamen Menge zusammen mit pharmazeutisch annehmbaren Träger- und Zusatzstoffen und gegebenenfalls noch eine oder mehrere andere pharmakologische Wirkstoffe in pharmakologisch wirksamen Mengen enthält.

**Patentansprüche für folgenden Vertragsstaat: ES**

1. Verfahren zur Herstellung von in 3-Stellung substituierten 3-(Thien-2-yl)-piperazin-2-onen der allgemeinen Formel I

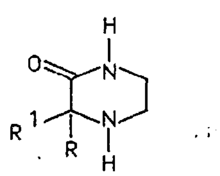

(I)

worin
R Phenyl; Phenyl mono-, di- oder tri-substituiert durch $(C_1\text{-}C_6)$Alkyl, $(C_1\text{-}C_6)$Alkoxy und/oder $(C_1\text{-}C_6)$Alkoxy-$(C_1\text{-}C_6)$alkyl; $(C_1\text{-}C_6)$Alkyl; Phenyl-$(C_1\text{-}C_6)$alkyl; Naphthyl-$(C_1\text{-}C_6)$alkyl; $(C_1\text{-}C_6)$Alkoxy-$(C_1\text{-}C_6)$alkyl; Di-$(C_1\text{-}C_6)$alkylamino-$(C_1\text{-}C_6)$alkyl und
$R^1$ 2-Thienyl
bedeuten, sowie ihrer pharmakologisch verträglichen Säureadditionsverbindungen, dadurch gekennzeichnet, daß das 3-(Thien-2-yl)-5,6-dihydro-(1H)-pyrazin-2-on der Formel II

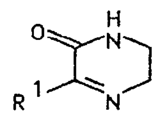

( I I )

mit einer metallorganischen Verbindung der allgemeinen Formel III

R - M (III)

worin M die Bedeutung von Li, $(CuLi)_{0,5}$, $Cd_{0,5}$, ZnHal, MgHal (Hal = I, Br oder Cl) und R die bereits angegebene Bedeutung besitzen, in einem inerten organischen Lösungsmittel oder Lösungsmittelgemisch im Temperaturbereich von 0°C bis zum Siedepunkt des Lösungsmittels oder Lösungsmittelgemischs umgesetzt, in üblicher Weise aufgearbeitet und gegebenenfalls in eine pharmakologisch annehmbare Säureadditionsverbindung überführt wird.

2. Verfahren nach Anspruch 1, dadurch gekennzeichnet, daß eine Verbindung der Formel III eingesetzt wird, bei der R Phenyl; Phenyl mono-, di- oder tri-substituiert durch $(C_1$-$C_4)$Alkyl, $(C_1$-$C_4)$Alkoxy und/oder $(C_1$-$C_4)$Alkoxy-$(C_1$-$C_4)$alkyl; $(C_1$-$C_4)$Alkyl; $(C_1$-$C_4)$Alkoxy-$(C_1$-$C_6)$alkyl; Phenyl-$(C_1$-$C_4)$alkyl; Naphthyl-$(C_1$-$C_4)$alkyl; Di-$(C_1$-$C_4)$alkylamino-$(C_1$-$C_4)$alkyl bedeutet.

3. Verfahren nach Anspruch 1 und/oder 2, dadurch gekennzeichnet, daß eine Verbindung der Formel III eingesetzt wird, bei der das für R stehende substituierte Phenyl monosubstituiert ist.

4. Verfahren gemäß Anspruch 1, dadurch gekennzeichnet, daß eine Verbindung der Formel III eingesetzt wird, bei der R $(C_1$-$C_4)$Alkyl bedeutet.

5. Verfahren gemäß Anspruch 1, dadurch gekennzeichnet, daß eine Verbindung der Formel III eingesetzt wird, bei der R Methyl, Ethyl, i-Propyl, Phenyl, Benzyl oder Phenethyl bedeutet.

6. Verfahren gemäß Anspruch 1, dadurch gekennzeichnet, daß eine Verbindung der Formel III eingesetzt wird, bei der R Methyl bedeutet.

7. Verfahren gemäß Anspruch 1, dadurch gekennzeichnet, daß eine Verbindung der Formel III eingesetzt wird, bei der R Phenyl bedeutet.

8. Verwendung von in 3-Stellung substituierten 3-(Thien-2-yl)-piperazin-2-onen der allgemeinen Formel I

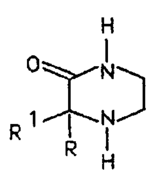

( I )

worin

R Phenyl; Phenyl mono-, di- oder tri-substituiert durch $(C_1$-$C_6)$Alkyl, $(C_1$-$C_6)$Alkoxy und/oder $(C_1$-$C_6)$Alkoxy-$(C_1$-$C_6)$alkyl; $(C_1$-$C_6)$Alkyl; Phenyl-$(C_1$-$C_6)$alkyl; Naphthyl-$(C_1$-$C_6)$alkyl; $(C_1$-$C_6)$Alkoxy-$(C_1$-$C_6)$alkyl; Di-$(C_1$-$C_6)$alkylamino-$(C_1$-$C_6)$alkyl und

$R^1$ 2-Thienyl

bedeuten, sowie ihrer pharmakologisch verträglichen Säureadditionsverbindungen als pharmakologische Wirkstoffe zur Herstellung pharmazeutischer Zubereitungen, die zur Verbesserung der Gehirnfunktion des Menschens und/oder zur Minderung der Folgen cerebraler Alterungsvorgänge des Menschens bestimmt sind.

**Patentansprüche für folgenden Vertragsstaat: GR**

1. In 3-Stellung substituierte 3-(Thien-2-yl)-piperazin-2-one der allgemeinen Formel I

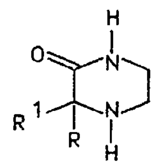

( I ) ·

worin

R Phenyl; Phenyl mono-, di- oder tri-substituiert durch $(C_1\text{-}C_6)$Alkyl, $(C_1\text{-}C_6)$Alkoxy und/oder $(C_1\text{-}C_6)$Alkoxy-$(C_1\text{-}C_6)$alkyl; $(C_1\text{-}C_6)$Alkyl; Phenyl-$(C_1\text{-}C_6)$alkyl; Naphthyl-$(C_1\text{-}C_6)$alkyl; $(C_1\text{-}C_6)$Alkoxy-$(C_1\text{-}C_6)$alkyl; Di-$(C_1\text{-}C_6)$alkylamino-$(C_1\text{-}C_6)$alkyl und

$R^1$ 2-Thienyl

bedeuten, sowie ihre pharmakologisch verträglichen Säureadditionsverbindungen.

2. Piperazinone nach Anspruch 1, dadurch gekennzeichnet, daß R Phenyl; Phenyl mono-, di- oder tri-substituiert durch $(C_1\text{-}C_4)$Alkyl, $(C_1\text{-}C_4)$Alkoxy und/oder $(C_1\text{-}C_4)$Alkoxy-$(C_1\text{-}C_4)$alkyl; $(C_1\text{-}C_4)$Alkyl; $(C_1\text{-}C_4)$Alkoxy-$(C_1\text{-}C_6)$alkyl; Phenyl-$(C_1\text{-}C_4)$alkyl; Naphthyl-$(C_1\text{-}C_4)$alkyl; Di-$(C_1\text{-}C_4)$alkylamino-$(C_1\text{-}C_4)$alkyl bedeutet.

3. Piperazinone nach Anspruch 1 und/oder 2, dadurch gekennzeichnet, daß das für R stehende substituierte Phenyl monosubstituiert ist.

4. Piperazinone gemäß Anspruch 1, dadurch gekennzeichnet, daß R $(C_1\text{-}C_4)$Alkyl bedeutet.

5. Piperazinone gemäß Anspruch 1, dadurch gekennzeichnet, daß R Methyl, Ethyl, i-Propyl, Phenyl, Benzyl oder Phenethyl bedeutet.

6. 3-Methyl-3-(thien-2-yl)-piperazin-2-on gemäß Anspruch 1 und seine pharmakologisch verträglichen Säureadditionsverbindungen.

7. 3-Phenyl-3-(thien-2-yl)-piperazin-2-on gemäß Anspruch 1 und seine pharmakologisch verträglichen Säureadditionsverbindungen.

8. Verfahren zur Herstellung der in einem oder mehreren der Ansprüche 1 bis 7 angegebenen Piperazin-2-on-verbindungen, dadurch gekennzeichnet, daß das 3-(Thien-2-yl)-5,6-dihydro-(1H)-pyrazin-2-on der Formel II

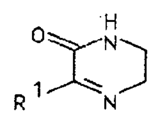

( I I )

mit einer metallorganischen Verbindung der allgemeinen Formel III

R - M (III)

worin M die Bedeutung von Li, $(CuLi)_{0,5}$, $Cd_{0.5}$, ZnHal, MgHal (Hal = I, Br oder Cl) und R die bereits angegebene Bedeutung besitzen, in einem inerten organischen Lösungsmittel oder Lösungsmittelgemisch im Temperaturbereich von 0°C bis zum Siedepunkt des Lösungsmittels oder Lösungsmittelgemischs umgesetzt, in üblicher Weise aufgearbeitet und gegebenenfalls in eine pharmakologisch annehmbare Säureadditionsverbindung überführt wird.

9. Verwendung der Piperazinone der Ansprüche 1 bis 7 und/oder ihre pharmakologisch annehmbaren Säureadditions verbindungen als pharmakologische Wirkstoffe zur Herstellung pharmazeutischer Zubereitungen, die zur Verbesserung der Gehirnfunktion des Menschens und/oder zur Minderung der Folgen cerebraler Alterungsvorgänge des Menschens bestimmt sind.

## Claims

### Claims for the following Contracting States: AT, BE, CH, DE, FR, GB, IT, LI, NL, SE

1. 3-(Thienyl-2-yl)-piperazin-2-ones, substituted in the 3-position, of the general formula I

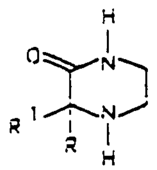

(I)

wherein R denotes phenyl; phenyl which is monosubstituted, disubstituted or trisubstituted by ($C_1$-$C_6$)-alkyl, ($C_1$-$C_6$)-alkoxy and/or ($C_1$-$C_6$)-alkoxy-($C_1$-$C_6$)-alkyl; ($C_1$-$C_6$)-alkyl; phenyl-($C_1$-$C_6$)-alkyl; naphthyl-($C_1$-$C_6$)-alkyl; ($C_1$-$C_6$)-alkoxy-($C_1$-$C_6$)-alkyl; or di-($C_1$-$C_6$)-alkylamino-($C_1$-$C_6$)-alkyl and $R^1$ denotes 2-thienyl, and to pharmacologically tolerable acid addition compounds thereof.

2. Piperazinones according to Claim 1, characterized in that R denotes phenyl; phenyl which is monosubstituted, disubstituted or trisubstituted by ($C_1$-$C_4$)-alkyl, ($C_1$-$C_4$)-alkoxy and/or ($C_1$-$C_4$)-alkoxy-($C_1$-$C_4$)-alkyl; ($C_1$-$C_4$)-alkyl; ($C_1$-$C_4$)-alkoxy-($C_1$-$C_6$)alkyl; phenyl-($C_1$-$C_4$)-alkyl; naphthyl-($C_1$-$C_4$)-alkyl; or di-($C_1$-$C_4$)-alkylamino-($C_1$-$C_4$)-alkyl.

3. Piperazinones according to Claim 1 and/or 2, characterized in that the phenyl represented by R is monosubstituted.

4. Piperazinones according to Claim 1, characterized in that R denotes ($C_1$-$C_4$)-alkyl.

5. Piperazinones according to Claim 1, characterized in that R denotes methyl, ethyl, isopropyl, phenyl, benzyl or phenethyl.

6. 3-Methyl-3-(thien-2-yl)-piperazin-2-one according to claim 1 and pharmacologically tolerable acid addition compounds thereof.

7. 3-Phenyl-3-(thien-2-yl)-piperazin-2-one according to claim 1 and pharmacologically tolerable acid addition compounds thereof.

8. Process for the preparation of the piperazin-2-one compounds indicated in one or more of Claims 1 to 7, characterized in that 3-(thien-2-yl)-5,6-dihydro-(1H)-pyrazin-2-one of the formula II

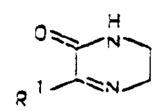

(II)

is reacted with an organometallic compound of the general formula III

R - M (III)

wherein M denotes Li, $(CuLi)_{0.5}$, $Cd_{0.5}$, ZnHal or MgHal (Hal = I, Br or Cl) and R has the meaning already indicated, in an inert organic solvent or solvent mixture within the temperature range from 0°C up to the boiling point of the solvent or solvent mixture, and the product is worked up in a customary manner and, if appropriate, is converted into a pharmacologically acceptable acid addition compound.

9. The use of the piperazinones of Claims 1 to 7 and/or pharmacologically acceptable acid addition compounds thereof as pharmacological active compounds for the preparation of pharmaceutical formulations intended for improving the human brain function and/or for reducing the consequences of cerebral aging processes in humans.

10. Pharmaceutical formulation, characterized in that it contains a compound of Claims 1 to 7 or a pharmacologically acceptable acid addition salt thereof as the active compound, in a pharmacologically effective amount, together with pharmaceutically acceptable excipients and additives and, if appropriate, also one or more other pharmacological active compounds in pharmacologically effective amounts.

**Claims for the following Contracting State: ES**

1. Process for preparing 3-(thienyl-2-yl)-piperazin-2-ones, substituted in the 3-position, of the general formula I

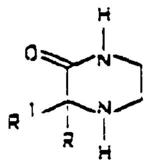

(I)

wherein R denotes phenyl; phenyl wich is monosubstituted, disubstituted or trisubstituted by ($C_1$-$C_6$)-alkyl, ($C_1$-$C_6$)-alkoxy and/or ($C_1$-$C_6$)-alkoxy-($C_1$-$C_6$)-alkyl; ($C_1$-$C_6$)-alkyl; phenyl-($C_1$-$C_6$)-alkyl; naphthyl-($C_1$-$C_6$)-alkyl; ($C_1$-$C_6$)-alkoxy-($C_1$-$C_6$)-alkyl; or di-($C_1$-$C_6$)-alkylamino-($C_1$-$C_6$)-alkyl and $R^1$ denotes 2-thienyl, and to pharmacologically tolerable acid addition compounds thereof, characterized in that 3-(thien-2-yl)-5,6-dihydro-(1H)-pyrazin-2-one of the formula II

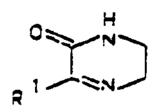

(II)

is reacted with an organometallic compound of the general formula III

R - M (III)

wherein M denotes Li, $(CuLi)_{0.5}$, $Cd_{0.5}$, ZnHal or MgHal (Hal = I, Br or Cl) and R has the meaning already indicated, in an inert organic solvent or solvent mixture within the temperature range from 0°C up to the boiling point of the solvent or solvent mixture, and the product is worked up in a customary manner and, if appropriate, is converted into a pharmacologically acceptable acid addition compound.

2. Process according to Claim 1, characterized in that a compound of formula III is employed wherein R denotes phenyl; phenyl which is monosubstituted, disubstituted or trisubstituted by ($C_1$-$C_4$)alkyl, ($C_1$-$C_4$)-alkoxy and/or ($C_1$-$C_4$)-alkoxy-($C_1$-$C_4$)-alkyl; ($C_1$-$C_4$)-alkyl; ($C_1$-$C_4$)-alkoxy-($C_1$-$C_6$)-alkyl; phenyl-($C_1$-$C_4$)-alkyl; naphthyl-($C_1$-$C_4$)-alkyl; or di-($C_1$-$C_4$)-alkylamino-($C_1$-$C_4$)-alkyl.

3. Process according to Claim 1 and/or 2, characterized in that a compound of formula III is employed wherein the phenyl represented by R is monosubstituted.

4. Process according to Claim 1, characterized in that a compound of formula III is employed wherein R denotes ($C_1$-$C_4$)-alkyl.

5. Process according to Claim 1, characterized in that a compound of formula III is employed wherein R denotes methyl, ethyl, isopropyl, phenyl, benzyl or phenethyl.

6. Process according to Claim 1, characterized in that a compound of formula III is employed wherein R denotes methyl.

7. Process according to Claim 1, characterized in that a compound of formula III is employed wherein R denotes phenyl.

8. Use of 3-(thienyl-2-yl)-piperazin-2-ones, substituted in the 3-position, of the general formula I

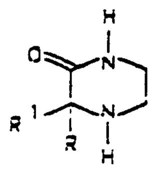

(I)

wherein R denotes phenyl; phenyl which is monosubstituted, disubstituted or trisubstituted by ($C_1$-$C_6$)-alkyl, ($C_1$-$C_6$)-alkoxy and/or ($C_1$-$C_6$)-alkoxy-($C_1$-$C_6$)-alkyl; ($C_1$-$C_6$)-alkyl; phenyl-($C_1$-$C_6$)-alkyl; naphthyl-($C_1$-$C_6$)-alkyl; ($C_1$-$C_6$)-alkoxy-($C_1$-$C_6$)-alkyl; or di-($C_1$-$C_6$)-alkylamino-($C_1$-$C_6$)-alkyl and $R^1$ denotes 2-thienyl, and to pharmacologically tolerable acid addition compounds thereof as the active compound, in a pharmacologically effective amount, together with pharmaceutically acceptable excipients and additives and, if appropriate, also one or more other pharmacological active compounds in pharmacologically effective amounts.

**Claims for the following Contracting State: GR**

1. 3-(Thienyl-2-yl)-piperazin-2-ones, substituted in the 3-position, of the general formula I

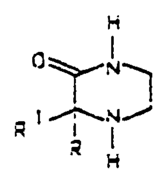

(I)

wherein R denotes phenyl; phenyl which is monosubstituted, disubstituted or trisubstituted by ($C_1$-$C_6$)-alkyl, ($C_1$-$C_6$)-alkoxy and/or ($C_1$-$C_6$)-alkoxy-($C_1$-$C_6$)-alkyl; ($C_1$-$C_6$)-alkyl; phenyl-($C_1$-$C_6$)-alkyl; naphthyl-($C_1$-$C_6$)-alkyl; ($C_1$-$C_6$)-alkoxy-($C_1$-$C_6$)-alkyl; or di-($C_1$-$C_6$)-alkylamino-($C_1$-$C_6$)-alkyl and $R^1$ denotes 2-thienyl, and to pharmacologically tolerable acid addition compounds thereof.

2. Piperazinones according to Claim 1, characterized in that R denotes phenyl; phenyl which is monosubstituted, disubstituted or trisubstituted by ($C_1$-$C_4$)-alkyl, ($C_1$-$C_4$)-alkoxy and/or ($C_1$-$C_4$)-alkoxy-($C_1$-$C_4$)-alkyl; ($C_1$-$C_4$)-alkyl; ($C_1$-$C_4$) -alkoxy-($C_1$-$C_6$)alkyl; phenyl-($C_1$-$C_4$)-alkyl; naphthyl-($C_1$-$C_4$)-alkyl; or di-($C_1$-$C_4$)-alkylamino-($C_1$-$C_4$)-alkyl.

3. Piperazinones according to Claim 1 and/or 2, characterized in that the phenyl represented by R is monosubstituted.

4. Piperazinones according to Claim 1, characterized in that R denotes ($C_1$-$C_4$)-alkyl.

5. Piperazinones according to Claim 1, characterized in that R denotes methyl, ethyl, isopropyl, phenyl, benzyl or phenethyl.

6. 3-Methyl-3-(thien-2-yl)-piperazin-2-one according to claim 1 and pharmacologically tolerable acid addition compounds thereof.

7. 3-Phenyl-3-(thien-2-yl)-piperazin-2-one according to claim 1 and pharmacologically tolerable acid addition compounds thereof.

8. Process for the preparation of the piperazin-2-one compound indicated in one or more of Claims 1 to 7, characterized in that 3-(thien-2-yl)-5,6-dihydro-(1H)-pyrazin-2-one of the Formula II

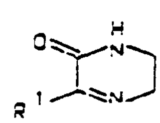

(II)

is reacted with an organometallic compound of the general formula III

R - M (III)

wherein M denotes Li, $(CuLi)_{0.5}$, $Cd_{0.5}$, ZnHal or MgHal (Hal = I, Br or Cl) and R has the meaning already indicated, in an inert organic solvent or solvent mixture within the temperature range from 0°C up to the boiling point of the solvent or solvent mixture, and the product is worked up in a customary manner and, if appropriate, is converted into a pharmacologically acceptable acid addition compound.

9. The use of the piperazinones of Claims 1 to 7 and/or pharmacologically acceptable acid addition compounds thereof as pharmacological active compounds for the preparation of pharmaceutical formulations intended for improving the human brain function and/or for reducing the consequences of cerebral aging processes in humans.

## Revendications

### Revendications pour les Etats contractants suivants: AT, BE, CH, DE, FR, GB, IT, LI, NL, SE

1. 3-(2-Thienyl)-pipérazine-2-ones substituées à la position 3 qui répondent à la formule générale I :

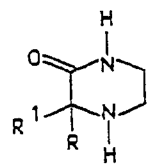

(I)

dans laquelle

R représente un phényle ; un phényle mono-, di- ou tri-substitué par un alkyle en $C_1$-$C_6$, un alcoxy en $C_1$-$C_6$ et/ou un ($C_1$-$C_6$)alcoxy-($C_1$-$C_6$)alkyle ; un alkyle en $C_1$-$C_6$; un phényl-($C_1$-$C_6$)alkyle ; un naphtyl-($C_1$-$C_6$)alkyle ; un ($C_1$-$C_6$)alcoxy-($C_1$-$C_6$)alkyle ; ou un di-($C_1$-$C_6$)alkylamino-($C_1$-$C_6$) alkyle et

$R^1$ représente un radical 2-thiényle, ainsi que les composé d'addition acceptables du point de vue pharmacologique qu'elles forment avec des acides.

2. Pipérazinones selon la revendication 1 caractérisées en ce que R représente un phényle ; un phényle mono-, di- ou tri-substitué par un alkyle en $C_1$-$C_4$, un alcoxy en $C_1$-$C_4$- et/ou un ($C_1$-$C_4$)alcoxy-($C_1$-$C_4$)alkyle ; un alkyle en $C_1$-$C_4$; un ($C_1$-$C_4$)alcoxy-($C_1$-$C_6$)alkyle ; un phényl-($C_1$-$C_4$)alkyle ; un naphtyl-($C_1$-$C_4$)alkyle ; ou un di-($C_1$-$C_4$)alkylamino-($C_1$-$C_4$)alkyle.

3. Pipérazinones selon la revendication 1 et/ou la revendication 2, caractérisées en ce que le phényle substitué que peut représenter R est monosubstitué.

4. Pipérazinones selon la revendication 1 caractérisées en ce que R représente un alkyle contenant de 1 à 4 atomes de carbone.

5. Pipérazinones selon la revendication 1 caractérisées en ce que R représente un radical méthyle, éthyle, isopropyle, phényle, benzyle ou phénéthyle.

6. 3-Méthyl-3-(2-thiényl)-pipérazine-2-one selon la revendication 1 et les composé d'addition pharmacologiquement acceptables qu'elle donne avec des acides.

7. 3-Phényl-3-(2-thienyl)-pipérazine-2-one selon la revendication 1 et les composé d'addition pharmacologiquement acceptables qu'elle donne avec des acides.

8. Procédé pour préparer les pipérazine-2-ones définies dans une ou plusieurs des revendications 1 à 7, procédé caractérisé en ce qu'on fait réagir la 3-(2-thienyl)-5,6-dihydro -(1H)-pyrazine-2-one de formule II :

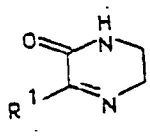

(II)

avec un composé organométallique répondant à la formule générale III :

R - M (III)

dans laquelle M représente Li, $(CuLi)_{0.5}$, $Cd_{0.5}$, ZnHal ou MgHal (Hal représentant I, Br ou Cl) et R a la signification qui lui a été donnée ci-dessus,

dans un solvant organique inerte ou un mélange de tels solvants, dans l'intervalle de température allant de 0 °C au point d'ébullition du solvant ou du mélange de solvants, on effectue le traitement complémentaire habituel et on transforme éventuellement en un composé d'addition d'acide acceptable du point de vue pharmacologique.

**9.** Application des pipérazinones selon les revendications 1 à 7 et/ou de leurs composé d'addition d'acides acceptables du point de vue pharmacologique comme substances actives pharmacologiques pour la fabrication de compositions pharmaceutiques destinées à améliorer le fonctionnement du cerveau chez l'homme et/ou à atténuer les suites de phénomènes cérébraux de sénescence chez l'homme.

**10.** Composition pharmaceutique caractérisée en ce qu'elle contient comme substance active, en une quantité pharmacologiquement efficace, un composé selon l'une quelconque des revendications 1 à 7, ou un sel d'un tel composé formé par addition avec un acide, ainsi que des excipients et additifs pharmacologiquement acceptables et, éventuellement, une ou plusieurs autres substances actives pharmacologiques, en des quantités pharmacologiquement efficaces.

**Revendications pour l'Etat contractant suivant: ES**

**1.** Procédé de préparation de 3-(2-thiényl)-pipérazine-2-ones substituées à la position 3 qui répondent à la formule générale I :

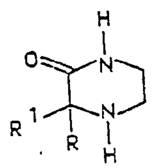

(I)

dans laquelle

R représente un phényle ; un phényle mono-, di- ou tri-substitué par un alkyle en $C_1$-$C_6$, un alcoxy en $C_1$-$C_6$ et/ou un $(C_1$-$C_6)$alcoxy-$(C_1$-$C_6)$alkyle ; un alkyle en $C_1$-$C_6$; un phényl-$(C_1$-$C_6)$alkyle ; un naphtyl-$(C_1$ -$C_6)$alkyle ; un $(C_1$-$C_6)$alcoxy-$(C_1$-$C_6)$alkyle ; ou un di-$(C_1$-$C_6)$alkylamino-$(C_1$-$C_6)$alkyle et

$R^1$ représente un radical 2-thiényle,

ainsi que des composés d'addition acceptables du point de vue pharmacologique qu'elles forment avec des acides, procédé caractérisé en ce qu'on fait réagir la 3-(2-thiényl)-5,6-dihydro-(1H)-pyrazine-2-one de formule II :

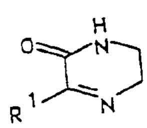

(II)

avec un composé organométallique répondant à la formule générale III :

R - M (III)

dans laquelle M représente Li, $(CuLi)_{0.5}$, $Cd_{0.5}$, ZnHal ou MgHal (Hal représentant I, Br ou Cl) et R a la signification qui lui a été donnée ci-dessus,

dans un solvant organique inerte ou un mélange de tels solvants, dans l'intervalle de température allant

de 0 °C au point d'ébullition du solvant ou du mélange de solvants, on effectue le traitement complémentaire habituel et on transforme éventuellement en un composé d'addition d'acide acceptable du point de vue pharmacologique.

2. Procédé selon la revendication 1 caractérisé en ce qu'on utilise un composé de formule III dans lequel R représente un phényle ; un phényle mono-, di- ou tri- substitué par un alkyle en $C_1$-$C_4$, un alcoxy en $C_1$-$C_4$ et/ou un ($C_1$-$C_4$)alcoxy-($C_1$-$C_4$)alkyle ; un alkyle en $C_1$-$C_4$ ; un ($C_1$-$C_4$)alcoxy-($C_1$-$C_4$)alkyle ; un phényl-($C_1$-$C_4$)alkyle ; un naphtyl-($C_1$-$C_4$)alkyle ; ou un di-($C_1$-$C_4$)alkylamino-($C_1$-$C_4$)alkyle.

3. Procédé selon la revendication 1 et/ou la revendication 2, caractérisé en ce que l'on utilise un composé de formule III dans lequel le phényle substitué que peut représenter R est monosubstitué.

4. Procédé selon la revendication 1 caractérisé en ce que l'on utilise un composé de formule III dans lequel R représente un alkyle contenant de 1 à 4 atomes de carbone.

5. Procédé selon la revendication 1 caractérisé en ce que l'on utilise un composé de formule III dans lequel R représente un radical méthyle, éthyle, isopropyle, phényle, benzyle ou phénéthyle.

6. Procédé selon la revendication 1 caractérisé en ce que l'on utilise un composé de formule III dans lequel R représente un méthyle.

7. Procédé selon la revendication 1 caractérisé en ce que l'on utilise un composé de formule III dans lequel R représente un phényle.

8. Application de 3-(2-thiényl)-pipérazine-2-ones substituées à la position 3 qui répondent à la formule générale I :

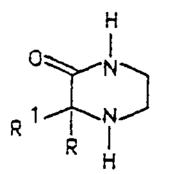

(I)

dans laquelle

R représente un phényle ; un phényle mono-, di- ou tri-substitué par un alkyle en $C_1$-$C_6$, un alcoxy en $C_1$-$C_6$ et/ou un ($C_1$-$C_6$)alcoxy-($C_1$-$C_6$)alkyle ; un alkyle en $C_1$-$C_6$ ; un phényl-($C_1$-$C_6$)alkyle ; un naphtyl-($C_1$-$C_6$)alkyle ; un ($C_1$-$C_6$)alcoxy-($C_1$-$C_6$)alkyle ; ou un di-($C_1$-$C_6$)alkylamino-($C_1$-$C_6$)alkyle et

$R^1$ représente un radical 2-thiényle,

ainsi que des composés d'addition acceptables du point de vue pharmacologique qu'elles forment avec des acides, comme substances actives pharmacologiques pour la fabrication de compositions pharmaceutiques destinées à améliorer le fonctionnement du cerveau chez l'homme et/ou à atténuer les suites de phénomènes cérébraux de sénescence chez l'homme.

**Revendications pour l'Etat contractant suivant: GR**

1. 3-(2-Thiényl)-pipérazine-2-ones substituées à la position 3 qui répondent à la formule générale I :

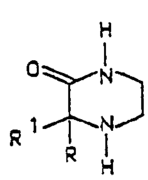

(I)

dans laquelle

R représente un phényle ; un phényle mono-, di- ou tri-substitué par un alkyle en $C_1$-$C_6$, un alcoxy en $C_1$-$C_6$ et/ou un ($C_1$-$C_6$)alcoxy-($C_1$-$C_6$)alkyle ; un alkyle en $C_1$-$C_6$ ; un phényl-($C_1$-$C_6$)alkyle ; un naphtyl-($C_1$-$C_6$)alkyle ; un ($C_1$-$C_6$)alcoxy-($C_1$-$C_6$)alkyle ; ou un di-($C_1$-$C_6$)alkylamino-($C_1$-$C_6$)alkyle et

$R^1$ représente un radical 2-thiényle,
ainsi que les composés d'addition acceptables du point de vue pharmacologique qu'elles forment avec des acides.

2. Pipérazinones selon la revendication 1 caractérisées en ce que R représente un phényle ; un phényle mono-, di-ou tri-substitué par un alkyle en $C_1$-$C_4$, un alcoxy en $C_1$-$C_4$ et/ou un ($C_1$-$C_4$)alcoxy-($C_1$-$C_4$)alkyle ; un alkyle en $C_1$-$C_4$; un ($C_1$-$C_4$)alcoxy-($C_1$-$C_6$)alkyle ; un phényl-($C_1$-$C_6$)alkyle ; un naphtyl-($C_1$-$C_4$)alkyle ; ou un di-($C_1$-$C_4$)alkylamino-($C_1$-$C_4$)alkyle.

3. Pipérazinones selon la revendication 1 et/ou la revendication 2, caractérisées en ce que le phényle substitué que peut représenter R est monosubstitué.

4. Pipérazinones selon la revendication 1 caractérisées en ce que R représente un alkyle contenant de 1 à 4 atomes de carbone.

5. Pipérazinones selon la revendication 1 caractérisées en ce que R représente un radical méthyle, éthyle, isopropyle, phényle, benzyle ou phénéthyle.

6. 3-Méthyl-3-(2-thiényl)-pipérazine-2-one selon la revendication 1 et les composés d'addition pharmacologiquement acceptables qu'elle donne avec des acides.

7. 3-Phényl-3-(2-thiényl)-pipérazine-2-one selon la revendication 1 et les composés d'addition pharmacologiquement acceptables qu'elle donne avec des acides.

8. Procédé pour préparer les pipérazine-2-ones définies dans une ou plusieurs des revendications 1 à 7, procédé caractérisé en ce qu'on fait réagir la 3-(2-thiényl)-5,6-dihydro-(1H)-pyrazine-2-one de formule II :

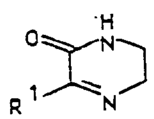

(II)

avec un composé organométallique répondant à la formule générale III :

R - M (III)

dans laquelle M représente Li, $(CuLi)_{0.5}$, $Cd_{0.5}$, ZnHal ou MgHal (Hal représentant 1, Br ou Cl) et R a la signification qui lui a été donnée ci-dessus,
dans un solvant organique inerte ou un mélange de tels solvants, dans l'intervalle de température allant de 0 °C au point d'ébullition du solvant ou du mélange de solvants, on effectue le traitement complémentaire habituel et on transforme éventuellement en un composé d'addition d'acide acceptable du point de vue pharmacologique.

9. Application des pipérazinones selon les revendications 1 à 7 et/ou de leurs composés d'addition d'acides acceptables du point de vue pharmacologique comme substances actives pharmacologiques pour la fabrication de compositions pharmaceutiques destinées à améliorer le fonctionnement du cerveau chez l'homme et/ou à atténuer les suites de phénomènes cérébraux de sénescence chez l'homme.